# EUROPEAN PATENT SPECIFICATION

(11) **EP 1 580 196 B1**
(45) Date of publication and mention of the grant of the patent: **04.08.2010**
(21) Application number: 03780713.8
(22) Date of filing: 11.12.2003
(51) Int. Cl.: C07K 19/00, C12N 15/62, C12Q 1/37

(54) **MONITOR PROTEIN FOR MEASURING PROCESSING OF PROTEIN**
ÜBERWACHUNGSPROTEIN ZUR MESSUNG DER PROTEINVERARBEITUNG
PROTEINE DE SURVEILLANCE DESTINEE A MESURER LE TRAITEMENT D'UNE PROTEINE

(30) Priority: 12.12.2002 JP 2002360744
(43) Date of publication of application: 28.09.2005
(73) Proprietor: Japan Science And Technology Agency, Kawaguchi-shi Saitama 332-0012 (JP)
(72) Inventor: OHMIYA, Yoshihiro, Ikeda-shi (JP); ASHITAKA, Emiko, Moriguchi-shi, Osaka 570-8506 (JP); ITO, Seiji, Moriguchi-shi, Osaka 570-8506 (JP)
(74) Representative: Smyth, Gyles Darren
(86) International application number: PCT/JP2003/015828
(87) International publication number: WO 2004/052934

(56) References cited:
- EP-A- 1 088 233
- EP-A- 1 265 990
- WO-A-00/20619
- WO-A2-02/25284
- HOULE BENOIT ET AL: "BRET2 (bioluminescence resonance energy transfer) monitoring of betaarrestin recruitment to agonist-stimulated GPCRs" FASEB JOURNAL, vol. 15, no. 4, 7 March 2001 (2001-03-07), page A219, XP008005390 & ANNUAL MEETING OF THE FEDERATION OF AMERICAN SOCIETIES FOR EXPERIMENTAL BIOLOGY ON EXPERIMENTAL BIOL; ORLANDO, FLORIDA, USA; MARCH 31-APRIL 04, 2001 ISSN: 0892-6638
- WAUD J P ET AL: "Measurement of proteases using chemiluminescence-resonance-energy-tra nsfer chimaeras between green fluorescent protein and aequorin." THE BIOCHEMICAL JOURNAL. 1 AUG 2001, vol. 357, no. Pt 3, 1 August 2001 (2001-08-01), pages 687-697, XP009066139 ISSN: 0264-6021
- KOHL TOBIAS ET AL: "A protease assay for two-photon crosscorrelation and FRET analysis based solely on fluorescent proteins." PROCEEDINGS OF THE NATIONAL ACADEMY OF SCIENCES OF THE UNITED STATES OF AMERICA. 17 SEP 2002, vol. 99, no. 19, 17 September 2002 (2002-09-17), pages 12161-12166, XP002380357 ISSN: 0027-8424
- TANAHASHI YUSUKE ET AL: "Continuous measurement of targeted promoter activity by a secreted bioluminescence reporter, Vargula hilgendorfii luciferase" ANALYTICAL BIOCHEMISTRY, vol. 289, no. 2, 15 February 2001 (2001-02-15), pages 260-266, XP002380358 ISSN: 0003-2697
- ASHIDAKA EMIKO: 'Tsukaku seigyo peptide.nocistatin no juyotai no cloning oyobi sansei kiko no kaimei' UEHARA MEMORIAL FOUNDATION KENKYU HOKOKUSHOSHU vol. 16, November 2002, pages 257 - 259, XP002903722
- OTSUJI TOMOMI ET AL.: 'Ichibunshinai BRET ni yoru processing kiko eno oyo' SEIKAGAKU vol. 4, no. 8, August 2002, page 1014, XP002903723
- PENNINGTON W.M. ET AL.: 'Synthesis of a fluorogenic Interleukin-1béta coverting enzyme substrate based on resonance energy transfer' PEPTIDE RESEARCH vol. 7, 1994, pages 72 - 76, XP002903724

## Description

### TECHNICAL FIELD

The present invention relates to a monitor protein comprising a region at which the protein is cleaved by processing and a property variable region in which an energy transfer property is changed by the cleavage, DNA encoding the same, and a method for screening a compound which facilitates or inhibits the processing.

### BACKGROUND ART

A protein which is a transcription product of a gene sometimes has a function directly, but in many cases, a protein which is a posttranslational product is activated through various modification processes such as sugar addition phosphorylation and processing. Among them, in a processing process, an active peptide is cut off from a preproprotein by being cleaved at a given site.

The processing process in which the active peptide is cut off from the preproprotein has been conventionally detected by an antibody specific for the active peptide after the cleavage or a direct change of molecular weights by electrophoresis or mass spectrometry. However, these methods are not simple, and are not procedures by which the processing is directly observed.

Light-emitting enzymes are effective as procedures to directly observe an intracellular transcription activity of a gene, and have been utilized as monitor proteins for detecting the gene expression. A fluorescent protein has a fluorescent activity without need of a cofactor nearly simultaneously with its intracellular expression. The fluorescent protein has been utilized as a monitor protein for intracellular localization of a protein using its fluorescent activity as an indicator.

A technique to track the changes of intermolecular or intramolecular interaction has been developed using energy transfer between a fluorescent protein and a fluorescent protein or a light-emitting enzyme and a fluorescent protein which are distinct proteins as an indicator. Monitor proteins have been constructed for analyzing the change of intracellular calcium using the energy transfer between the fluorescent protein and the fluorescent protein as the indicator and for analyzing phosphorylation of the protein using the energy transfer between the light-emitting enzyme and the fluorescent protein as the indicator. However, there is no available monitor protein which visualizes and quantifies the processing process using the energy transfer as the indicator.

It is an object of the present invention to develop a monitor protein capable of visualizing the processing process, a method for optimization thereof, a method for screening processing enzymes using the monitor protein and a method for screening medicaments which inhibit or facilitate a processing enzyme activity as well as utilize them for the treatment of diseases, examinations and new drug development.

### DISCLOSURE OF THE INVENTION

If a monitor protein construct where a processing region is inserted between a light-emitting enzyme and a fluorescent protein which can transfer energy is made, energy transfer is changed before and after the processing and a luminescence spectrum is also changed therewith. By the use of this spectral change as an indicator, it is possible to quantify an active peptide after the processing, quantify a processing enzyme activity and analyze the localization of a processing site, further screen a gene and a protein which control the processing enzyme activity as well as it becomes possible to utilize them for the treatment of various diseases and new drug development (development of medicaments which facilitate or inhibit the processing) using the processing as the indicator.

As a result of an extensive study for solving the above problems, the present inventor has made a monitor protein which has a processing cleavage region cleaved by the processing and a property variable region which exhibits a change in luminescent and fluorescent energy property by the processing.

The present invention relates to the following monitor proteins, DNA encoding the same, and methods for screening compounds which facilitate or inhibit the processing.
[1] A monitor protein, capable of measuring processing of a protein, comprising:
   - one or more processing cleavage regions containing an amino acid residue or an amino acid sequence to be processed; and
   - one or more property variable regions, each comprising a luminescent protein and a fluorescent protein which exhibit changes in their luminescent and fluorescent energy properties upon said processing, characterised in that:
      the luminescent protein is the *Cypridina noctiluca* secretory luciferase; and the fluorescent protein is the luminescent *Aequorea victoria* yellow fluorescent protein.
[2] The monitor protein according to [1] wherein the monitor protein is a secretory protein.
[3] The monitor protein according to [1] wherein a processing enzyme which cleaves the processing cleavage region is any one selected from the group consisting of PC1, PC2, furin, proteasome, cathepsin and thrombin.
[4] The monitor protein according to [3] wherein the processing enzyme is PC1.
[5] The monitor protein according to [1] wherein the processing cleavage region is located between the luminescent protein and the fluorescent protein which constitute the property variable region.
[6] The monitor protein according to [1] wherein the processing cleavage region is composed of a sequence of 6 to 100 amino acids including a cleavage point cleaved by the processing enzyme.
[7] A monitor protein according to [1] having the amino acid sequence represented in SEQ. ID NO. 2 or a mutant thereof encoded by a DNA sequence having at least 90% sequence homology to SEQ. ID NO. 1, wherein the mutant protein comprises:
   one or more processing cleavage regions containing an amino acid residue or an amino acid sequence to be processed; and
   one or more property variable regions, each comprising a luminescent protein and a fluorescent protein which exhibit changes in their luminescent and fluorescent energy properties upon said processing.
[8] The monitor protein according to [1] wherein the processing cleavage region is SEQKQLQKRFGGFTGG.
[9] DNA encoding the monitor protein according to any of [1] to [8].
[10] DNA according to [8[, having the sequence according to SEQ. ID NO. 1 or DNA which:
   hybridizes to said sequence under stringency conditions of 1x SSC and 0.1% SDS at 37 degrees C; or
   has at least 90% sequence homology with SEQ. ID NO. 1, wherein the DNA encodes a protein comprising:
      one or more processing cleavage regions containing an amino acid residue or an amino acid sequence to be processed; and
      one or more property variable regions, each comprising a luminescent protein and a fluorescent protein which exhibit changes in their luminescent and fluorescent energy properties upon said processing.
[11] An expression vector comprising the DNA according to [9].
[12] A method for measuring a processing ability of a certain cell characterized by introducing any of the monitor protein according to any of [1] to [8], the DNA according to [9] or [10] and the expression vector according to [11] into the cell, and quantitatively evaluating a change in energy transfer property of the monitor protein.
[13] The method according to [12] wherein said cell is a cell derived from a human.
[14] A method for measuring a processing ability of a subject protein comprising a step of reacting the subject protein with the monitor protein according to any of [1] to [8] and a step of measuring a change in energy transfer property of the monitor protein.
[15] A method for screening a processing enzyme, comprising a step of reacting a subject protein with the monitor protein according to any of [1] to [8] and a step of selecting the subject protein which changes a property of the monitor protein by measuring a change in energy transfer property of the monitor protein before and after the reaction with the subject protein.
[16] A method for screening a compound which facilitates or inhibits processing, comprising a step of contacting a subject sample and a processing enzyme and the monitor protein according to any of [1] to [8] and a step of selecting a sample which facilitates or inhibits the property by measuring a change in energy transfer property of the monitor protein before and after the contact with the subject sample.
[17] A method for screening a compound which facilitates or inhibits processing (enzyme activity), comprising a step of preparing a cell into which any of the monitor protein according to any of [1] to [8], the DNA according to [9] or [10] and the expression vector according to [11] is introduced, and a step of selecting a sample which facilitates or inhibits a property change by measuring the property change in energy transfer of the monitor protein expressed in the cell in the presence or absence of the subject sample.

The present specification further discloses the following invention.
1. Proteins showing in the following (1) to (4):
   (1) a monitor protein represented by an amino acid sequence in SEQ ID NO:2;
   (2) a monitor protein wherein a sequence or a position in a region cleaved by processing and a property variable region (a luminescent protein and a fluorescent protein) is changed in the amino acid sequence in SEQ ID NO:2;
   (3) a monitor protein wherein a processing cleavage region figured by an underline in the amino acid sequence in SEQ ID NO:2 (amino acid sequence of 18 amino acid residues including a cleavage point of prepronocistatin protein) is substituted with 10 to 100 amino acid residues, preferably 20 to 40 amino acid residues in the vicinity of a processing cleavage site; and
   (4) a monitor protein wherein a property is variable in a property variable region due to a change of a three dimensional structure in the vicinity of the processing cleavage region. The monitor protein wherein a luminescent protein and a fluorescent protein are arranged with sandwiching the processing region, the energy transfer from the luminescent protein to the fluorescent protein is not effected before and after the processing, and thus a luminescent color is changed is suitable.

In a preferred embodiment, in the monitor protein, the luminescent protein and the fluorescent protein are arranged at both termini of the property variable region, respectively. In the monitor protein, either one may be present at the N-terminus, but it is more preferable that the luminescent protein and the fluorescent protein are present in this order from the N-terminus. In the monitor protein having the luminescent protein and the fluorescent protein, when the processing does not occur, the monitor protein emits yellow-green light by the energy transfer of blue light emitted from the luminescent protein (secretory *Cypridina noctiluca,* light-emitting enzyme) to the fluorescent protein mutant (EYFP). When the processing has occurred, the energy transfer is not effected and only the blue light is emitted from the monitor protein. The processing can be analyzed using this color change of the emitted light in the property variable region as the indicator.

Conventionally, there have been monitor proteins with which intracellular phosphorylation and calcium dynamics can be observed. However, the present inventor has demonstrated a monitor protein capable of visualizing and quantifying the processing process for the first time. A system for analyzing cellular functions has been designed using the change in the energy transfer between the fluorescent protein and the fluorescent protein or between the luminescent protein and the fluorescent protein as the indicator. However, the present researcher has utilized it for a system which analyzes the processing for the first time.

The present invention will be described in more detail below.

The monitor protein of the present invention is characterized by comprising the processing cleavage region and the property variable region.

The processing cleavage region is a region comprising two or more amino acids, which is cleaved by the processing enzyme, and the amino acid sequence thereof is changed depending on a type of the processing enzyme subjected to be monitored.

In the present invention, the processing cleavage region is the region comprising the amino acid sequence capable of being recognized and cleaved by the processing enzyme, comprises one or more amino acids, and the amino acid sequence thereof is changed depending on the type of the processing enzyme subjected to be monitored. For example, in the monitor protein represented by SEQ ID NO:2, PC1, which is the processing enzyme, recognizes and cleaves a cleavage point, "KR", but the cleavage occurs at only the processing cleavage region although there are four "KR" in SEQ ID NO:2 (two in a luciferase moiety, one in the processing cleavage region, and one in YFP). Therefore, the processing cleavage region may comprise not only the cleavage point (KR) but also further an additional sequence having an auxiliary portion for the enzyme to recognize and be able to cleave the cleavage point, which scarcely or does not affect the cleavage at all. One example of the processing cleavage region includes "SEQKQLQKRFGGFTGG" for the processing enzyme (PC1).

The processing cleavage region will be illustrated with reference to PC1 as the processing enzyme.

The cleavage recognition sequence of PC1 is Lys-Arg, and the processing cleavage region contains Lys-Arg and further links 3 to 50, preferably 4 to 40 and more preferably 5 to 20 amino acids at the C-terminus and the N-terminus. The amino acids used for the processing cleavage region are preferably usual amino acids such as Gly, Ala, His, Arg, Lys, Ser, Cys (including cystine), Thr, Met, Phe, Tyr, Trp, Leu, Ile, Val, Glu, Asp, Gln, Asp, Pro which constitute proteins, but may be other natural or synthetic amino acids such as Sar, β-alanine, norvaline, ornithine and cysteic acid.

The above is the case where the processing enzyme is PC1, and the processing cleavage region for the other processing enzyme can be also designed in a similar manner.

The total number of the amino acids in the cleavage region is about 6 to 100, preferably about 8 to 50, and more preferably about 10 to 40.

The processing enzyme is not particularly limited as long as it exclusively degrades a protein precursor synthesized in vivo and converts into a mature protein, and for example, PC1, PC2, furin, proteasome, cathepsin and thrombin are exemplified.

The processing enzymes and the cleavage regions thereof in addition to PC1 are exemplified below.

**Table 1**

| Processing enzyme | Amino acid sequence at cleavage point of processing |
|---|---|
| PC1 | KR↓ |
| PC2 | KR↓ |
| Furin | RRKR↓ |
| Proteasome | KM↓ |
| Cathepsin | KM↓ |
| Thrombin | LVPR↓ |

For example, PC1, PC2 and furin cleave a C-terminal protein of KR, and a particular processing enzyme becomes possible to cleave by changing a structure of the sequence in the vicinity of KR or a structure of a whole protein.

When there is one processing enzyme to be monitored, one cleavage recognition sequence may be included. When multiple processing enzymes are simultaneously monitored, it is possible to introduce different cleavage recognition sequences corresponding to two or more processing enzymes into the cleavage region. A compound which regulates only a particular processing enzyme activity can be screened by constructing the cleavage region having only the cleavage recognition sequence of the particular processing enzyme and having no cleavage recognition sequence of the other processing enzyme. Even when one processing enzyme which acts upon multiple substrates is monitored, a compound which facilitates or inhibits the processing of the particular substrate can be screened by selecting the cleavage recognition sequence or the sequence in the vicinity thereof in accordance with the sequence of the particular substrate.

In the property variable region, the energy transfer property may be changed when cleaved by the processing enzyme, The property variable region in accordance with the invention comprises a specific luminescent protein and a specific fluorescent protein as are described below. Also disclosed herein is a property variable region comprising a luminescent protein and a colored protein.

When the luminescent protein and the fluorescent protein are linked through the processing cleavage region, before the processing region is cleaved, the energy transfer from the luminescent protein to the fluorescent protein occurs, but after being cleaved, this energy transfer does not occur. Thus, it is advantageous in that the presence or absence of the cleavage can be easily quantified by measuring the energy transfer. It is preferable that the luminescent protein and the processing cleavage region and the fluorescent protein are linked in the order of the luminescent protein-the processing cleavage region-the fluorescent protein, but may be linked in the order of the fluorescent protein-the processing cleavage region-the luminescent protein as long as a luminescent/fluorescent property is changed by the cleavage of the processing enzyme.

The processing cleavage region may also be introduced into a site which does not crucially affect the luminescent property in the luminescent protein. In this case, the luminescent protein may lose the luminescent property after the processing.

Likewise, the processing cleavage region may also be introduced into a site which does not crucially affect the fluorescent property in the fluorescent protein. In this case, the fluorescent protein may lose the fluorescent property after the processing.

As the luminescent protein, luciferases derived from various luminescent organisms such as *Cypridina noctiluca, Acanthephya purpurea,* luminescent insects (firefly, headlight beetle, etc.), luminescent earthworm, *Latia neritoides, Renilla* and *Aequorea victoria* (aequorin) are disclosed herein. In accordance with the invention, the luminescent protein is the *Cypridina noctiluca* luciferase which is a secretory type, and is used because the monitor protein also becomes secretory when this is used. When the secretory monitor protein is used, a level of the processing which occurs in living cells can be evaluated without disrupting the cells. In the case of non-secretory luciferase such as luciferase derived from *Renilla,* it can be utilized as a secretory luminescent protein by introducing a secretory protein into the N-terminal side.

As the fluorescent protein, a green fluorescent protein (GFP), a yellow fluorescent protein (YFP), a blue fluorescent protein (BFP), a cyan fluorescent protein (CFP), DsRED and a red fluorescent protein (RFP) are disclosed herein. In accordance with the invention, the luminescent protein is the luminescent *Aequorea victoria* yellow fluorescent protein. As the colored protein, a colored blue protein (phycocyanin) and a red colored protein (phycoerythrin) are disclosed herein. Phycocyanin and phycoerythrin can absorb the light of firefly luciferase.

The fluorescent protein disclosed herein is selected so that the light released from the biological luminescent protein is an excitation wavelength of the fluorescent protein. Such a combination includes, for example, the following.

**Table 2**

| Biological luminescent protein | Fluorescent protein or colored protein |
|---|---|
| *Cypridina noctiluca* luciferase | GFP, YFP, BFP, CFP, DsRED, RFP |
| Firefly luciferase | DsRED Phycocyanin, Phycoerythrin |
| Luminescent *Pyrocystis lunula* luciferase | GFP, YFP, BFP, CFP, DsRED, RFP |
| Headlight beetle luciferase | DsRED |
| *Renilla* luciferase | GFP, YFP, BFP, CFP, DsRED, RFP |
| Aequorin | GFP, YFP, BFP, CFP, DsRED, RFP |

The proteins shown in the following (1) to (3) are also disclosed herein.
(1) The monitor protein represented by the amino acid sequence of SEQ ID NO. 2.
(2) The monitor protein represented by the amino acid sequence having one or more amino acid deletions, substitutions or additions in the amino acid sequence in SEQ ID NO. 2, and which has the light-emitting enzyme activity, the fluorescent protein activity and the energy transfer activity.
(3) The monitor protein encoded by the DNA represented by the base sequence in SEQ ID NO:1 or the DNA which hybridizes with the DNA complementary thereto under the stringent condition, and which has the light-emitting enzyme activity, the fluorescent protein activity and the energy transfer activity.

The protein (2) is the protein where mutations to an extent that the light-emitting enzyme activity, the fluorescent protein activity and the energy transfer activity are not lost are introduced into the protein (1). Such mutations naturally occur and further encompass artificial mutations. Procedures by which induce the artificial mutation include, but are not limited to site-directed mutagenesis (Nucleic Acids Res., 10:6487-6500, 1982). The number of mutated amino acids are not limited as long as the secretion, the luminescence, the fluorescent activity and the energy transfer property are not lost, and is typically within 20 amino acids, preferably within 15 amino acids, more preferably within 10 amino acids and most preferably within 5 amino acids. Whether the protein into which the mutation is introduced keeps the luminescent/fluorescent activity can be determined by examining the luminescent/fluorescent activity of the protein.

The protein (3) is the monitor protein obtained by utilizing the hybridization of DNA, and which has the secretory type light-emitting enzyme activity, the fluorescent protein activity and the energy transfer activity. The "stringent condition" in the protein (3) refers to the condition where only the specific hybridization occurs whereas no non-specific hybridization occurs. Such a condition is typically the condition of "1 x SSC and 0.1% SDS at 37°C", preferably "0.5 x SSC and 0.1% SDS at 42°C", and more preferably "0.2 x SSC and 0.1% SDS at 65°C". The DNA obtained by the hybridization typically has high homology to the DNA represented by the base sequence in SEQ ID NO:1. The high homology indicates 60% or more homology, preferably 75% or more homology and more preferably 90% or more homology.

The protein of the present invention can be obtained by incorporating a gene of the present invention described later into an expression vector and expressing it in appropriate host cells. As the expression vector, for example, pBT-VL-b-YFP and the like can be used. As the host cell, *Escherichia coli* BL21 strain and the like can be used.

Genes disclosed herein include the genes represented by the following (1) and (2).
(1) The gene represented by the base sequence in SEQ ID NO:1.
(2) The DNA sequence which hybridizes with the DNA represented by the base sequence in SEQ ID NO:1 or the DNA complementary thereto under the stringent condition.

The protein encoded by the above DNA is the protein obtained by utilizing the hybridization of DNA one another, and which has the secretory type light-emitting enzyme activity, the fluorescent protein activity and the energy transfer activity.

The "stringent condition" herein refers to the condition where only the specific hybridization occurs whereas no non-specific hybridization occurs. Such a condition is typically the condition of "1 x SSC and 0.1% SDS at 37°C", preferably "0.5 x SSC and 0.1% SDS at 42°C", and more preferably "0.2 x SSC and 0.1% SDS at 65°C". The DNA obtained by the hybridization typically has high homology to the DNA represented by the base sequence in SEQ ID NO:1 or 2. The high homology indicates 60% or more homology, preferably 75% or more homology, more preferably 90% or more homology, and particularly 95% or more homology.

The protein of the present invention can be obtained by incorporating the gene of the present invention described later into the expression vector and expressing it in appropriate host cells. As the expression vector, for example, pBT-VL-mp-YFP (VL, mp and YFP indicate *Cypridina noctiluca* luciferase, a monitor peptide and the luminescent Aequorea victoria yellow fluorescent protein, respectively) and the like can be used. The host cells include eukaryotic cells such as mammalian cells and yeast cells, and prokaryotic cells such as *Escherichia coli, Bacillus subtilis*, algal and fungal cells, and any of them may be used. As the preferable host cell, mammalian cultured cell COS7 cell line (in this system, it is important to go through protein synthesis and protein modification processes of a mammalian system, i.e., this process will be monitored) and the like can be used.

A gene (polynucleotide) encoding a preferable chimera protein of the present invention is
(1) the gene having the base sequence in SEQ ID NO:1 or
(2) the gene having the DNA represented by the base sequence in SEQ ID NO:1 or the DNA which hybridizes with the DNA complementary thereto under the stringent condition.

### (Screening method I)

The change in the energy transfer property of the monitor protein is quantitatively evaluated by introducing any of the monitor protein, the DNA sequence or the expression vector into certain cells.

The method enables one to quantitatively evaluate the processing ability of the cell.

### (Screening method II)

The processing ability of a subject protein is quantitatively evaluated by reacting the subject protein with the monitor protein of the present invention and measuring the change in the energy transfer property of the monitor protein.

In the method, what processing enzyme the subject protein is or what processing enzyme activity is strong can be quantitatively measured by typically preparing multiple monitor proteins and analyzing which monitor protein has its energy transfer property changed by the subject protein.

### (Screening method III)

A new processing enzyme can be screened by performing a step of reacting a subject protein with the monitor protein of the present invention and a step of selecting the subject protein which changes the property of the monitor protein by measuring the change in the energy transfer property of the monitor protein before and after the reaction with the subject protein.

### (Screening method IV)

The method for screening a compound which facilitates or inhibits the processing in a cell free system, comprises the step of contacting a subject sample with the processing enzyme and the monitor protein of the present invention, and the step of selecting the sample which facilitates or inhibits the property by measuring the change in the energy transfer property of the monitor protein before and after the contact with the subject sample.

By this method, the compound which facilitates or inhibits the processing can be screened without considering factors such as migration into cells.

The method for screening a compound which facilitates or inhibits the processing (enzyme activity), comprises the step of preparing cells into which any of the monitor protein, the DNA sequence encoding the monitor protein and the expression vector comprising the DNA sequence of the present invention has been introduced, and the step of selecting the sample which facilitates or inhibits the property by measuring the change in the energy transfer property of the monitor protein expressed in the cells in the presence or absence of the subject sample.

By this method, the compound which exerts medicinal efficacy by migrating into blood by oral administration can be screened.

The present invention provides the monitor protein capable of quantifying the novel processing, the gene encoding the same, and the gene which controls the expression of the present enzyme. By the use of the present monitor protein, the processing process which occurs in the cell can be visualized without disrupting the cell. These can be utilized for the treatment of diseases, the examination and the new drug development.

### BRIEF DESCRIPTION OF THE DRAWINGS

Fig. 1 shows constructs where a processing site (A), a mutated processing site (B) or deleted processing site (C) is inserted into an energy transferable secretory chimera protein.
Fig. 2 shows DNA sequences of regions where a processing site (A), a mutated processing site (B) or deleted processing site (C) is inserted into an energy transferable secretory chimera protein.
Fig. 3 shows Western blotting analysis of intracellular and extracellular samples of cells into which a secretory chimera protein where a processing sequence had been inserted was introduced, and detection results of changes in cleavage efficiency when a processing enzyme was introduced.
Fig. 4 shows that energy transfer efficiency and spectra are changed when a processing enzyme was introduced into cells which expressed a secretory chimera protein where a processing sequence had been inserted. (a) Vluc-NST/Nco-EYFP alone, (b) PC1 was expressed with Vluc-NST/Nco-EYFP, (c) PC2 was expressed with Vluc-NST/Nco-EYFP, and (d) Vluc.
Fig. 5 shows that energy transfer efficiency of a secretory chimera protein where a processing sequence had been inserted was largely reduced by a cleavage enzyme PC1, and also shows results of cleavage efficiency quantified by the results of the Western blotting.

Next, the present invention will be described in more detail with reference to the following Examples.

### BEST MODE FOR CARRYING OUT THE INVENTION

The present invention will be described in more detail using the following Examples.

### Example 1

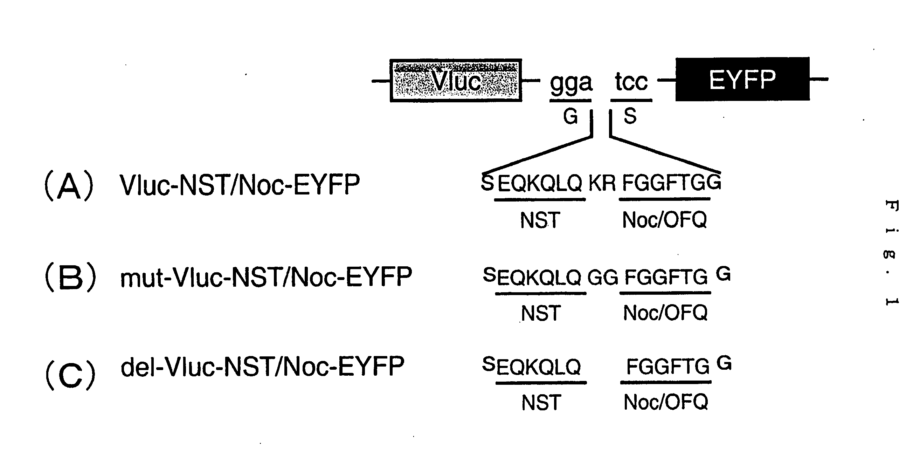

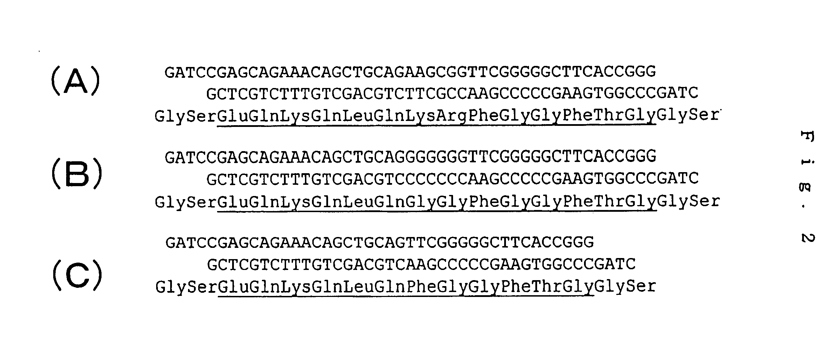

Nocistatin and nociceptin are hormone peptides cleaved off from the same preproprotein by a cleavage enzyme in the processing process, and are known to control reciprocal effects when pain runs in the body. A monitor protein where a nocistatin (described as NST in the figure) peptide sequence and a nociceptin (described as Noc in the figure) peptide sequence to be processed had been inserted in an insertion site of a secretory chimera protein where energy was transferable between a luminescent protein and a fluorescent protein was made (Fig. 1 (A)). In conjunction therewith, a monitor protein where a peptide sequence Lys-Arg which was essential for cleavage recognition and was cleaved had been mutated to Gly-Gly (Fig. 1 (B)), and a monitor protein where the peptide sequence was deleted (Fig, 1 (C)) were made. They were referred to as Vluc-NST/Noc-EYFP, mut-Vluc-NST/Noc-EYFP and del-Vluc-NST/Noc-EYFP, respectively. A BamHI site which was an insertion restriction enzyme site of the monitor protein was cleaved, and subsequently chemically synthesized DNA sequences shown in Fig. 2 were ligated by a ligase reaction. The insertion was confirmed by DNA sequencing.

### Example 2

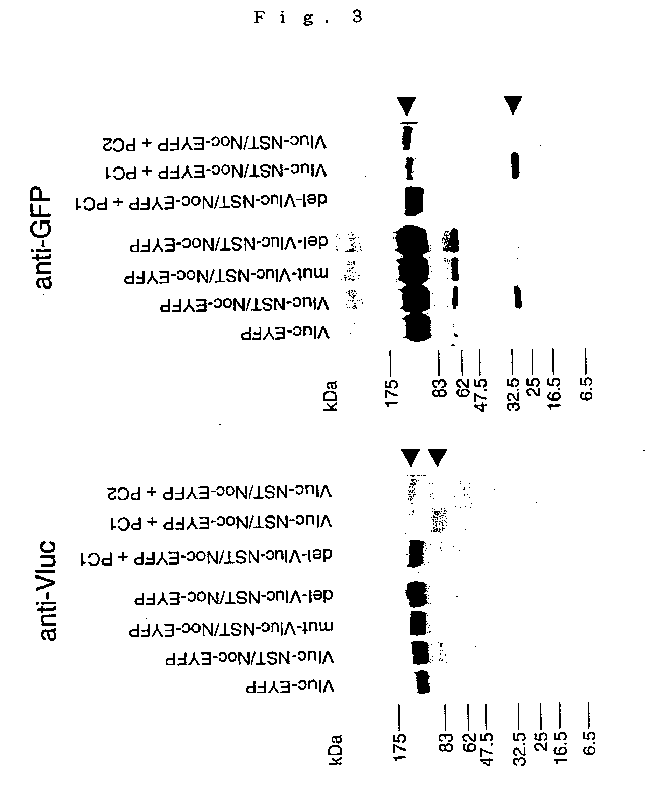

NG108-15 cells derived from nervous cells were transfected with gene vectors containing three monitor proteins, Vluc-NST/Noc-EYFP, mut-Vluc-NST/Noc-EYFP and del-Vluc-NST/Noc-EYFP using a commercially available transfection reagent. Proteins secreted in culture media were analyzed by a Western blotting method (Fig. 3). Vluc-EYFP which had no insertion sequence was used as a control. An anti-*Cypridina noctiluca* luciferase antibody (ant-Vluc) recognizes a light-emitting enzyme at the N-terminal side of the monitor protein, and four types of the monitor proteins have a molecular weight of 95 kDa. An anti-green fluorescent protein antibody (anti-GFP) recognizes the fluorescent protein at the C-terminal side of the monitor protein, and four monitor proteins showed the molecular weight of 95 kDa as a main protein. However, in the monitor protein of Vluc-NST/Noc-EYFP, a minor band at 27 kDa compared to the band at 95 kDa was observed. A processing enzyme was expressed in NG108-15 cells derived from the nervous cells, and cleaved the monitor protein, the antibody recognized EYFP at the C-terminal side after the cleavage and consequently this band was produced. In the mutated or deleted, two mut-Vluc-NST/Noc-EYFP and del-Vluc-NST/Noc-EYFP, this 27 kDa band was not detected and it has been demonstrated that the processing enzyme can strictly recognize the inserted amino acid sequence. But, a 68 kDa band recognized by anti-GFP seems to be artificial. In order to demonstrate that the cleavage which occurs in the monitor protein of Vluc-NST/Noc-EYFP is attributed to either PC1 or PC2 which is a representative processing enzyme which cleaves insulin and enkephalin, PC1 or PC2 was forcibly expressed in the cells into which the monitor protein had been transfected. As a result, it has been demonstrated that PC1 remarkably increased the 27 kDa band compared to the 95 kDa band whereas PC2 scarcely changed. Consequently, it has been demonstrated for the first time that the processing between nocistatin and nociceptin (NST/Noc) occurs due to PC1.

### Example 3

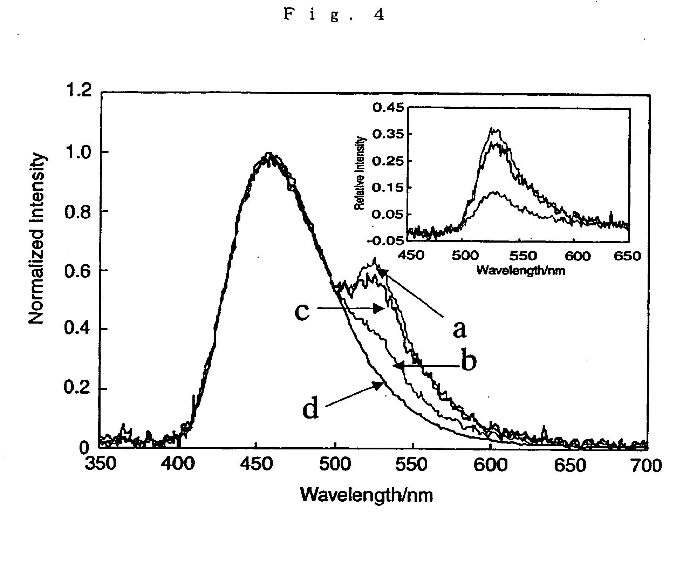

NG108-15 cells derived from nervous cells were transfected with gene vectors containing the monitor protein, Vluc-NST/Noc-EYFP and Vluc using a commercially available transfection reagent. In conjunction therewith, the processing enzyme, PC1 or PC2 was forcibly expressed in the cells which had expressed the monitor protein, Vluc-NST/Noc-EYFP. Luminescence spectra in culture media secreted by these four types of the cells were measured (Fig. 4). In the monitor protein, Vluc-NST/Noc-EYFP, a peak at 525 nm caused by the energy transfer which had occurred in the monitor protein could be observed in addition to a peak at 460 nm conformed to that of the luminescence spectrum from Vluc alone. In the culture medium of the cells where PC2 had been expressed in this monitor protein, Vluc-NST/Noc-EYFP, the spectrum was not changed whereas in the culture medium of the cells where PC1 had been expressed in Vluc-NST/Noc-EYFP, the peak at 525 nm was reduced. This reveals that the processing enzyme PC1 cleaved the monitor protein, Vluc-NST/Noc-EYFP and the energy transfer in the monitor protein did not occur as is also evident from the result of the Western blotting. That is, by measuring the luminescence spectrum in the culture medium, the processing level which occurs in the living cells can be evaluated without disrupting the cells.

### Example 4

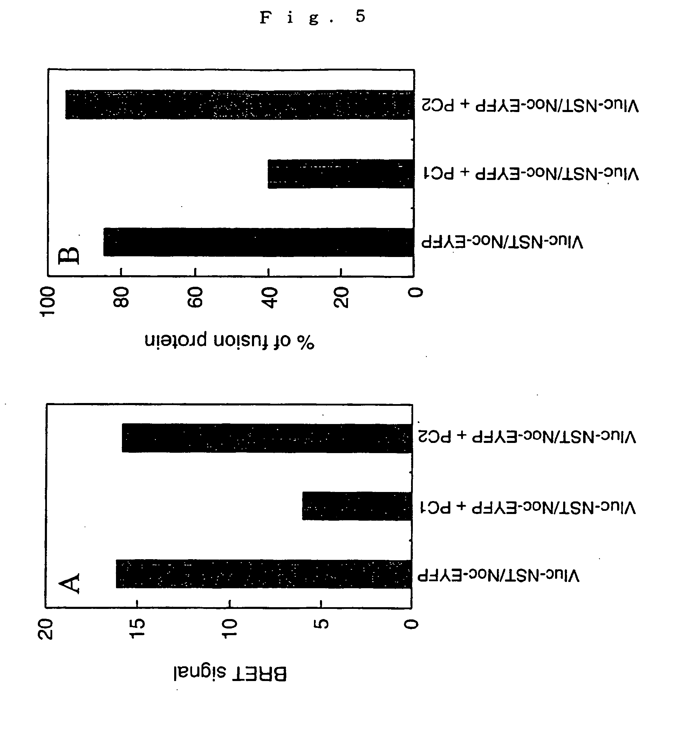

The cleavage efficiency of the monitor protein, Vluc-NST/Noc-EYFP transfected into NG108-15 cells derived from the nervous cells was detected by the Western blotting, and a mass of non-cleaved protein was quantified by an image analysis (Fig. 5B). Subtracted spectra were obtained by comparing the luminescence spectra of the monitor protein, Vluc-NST/Noc-EYFP with the spectrum of Vluc alone (inner figure in Fig. 4), and spectral areas were evaluated as energy transfer effects (BRET signal)(Fig. 5A). In the cases of the monitor protein, Vluc-NST/Noc-EYFP and the monitor protein, Vluc-NST/Noc-EYFP in which PC2 had been introduced, both the non-cleaved protein mass and the BRET signal shows that the cleavage did not occur. Meanwhile, in the case of the monitor protein, Vluc-NST/Noc-EYFP in which PC1 had been introduced, both the non-cleaved protein mass and the BRET signal shows that 60% monitor protein was cleaved. This reveals that the efficiency of the processing which occurs in the cells can be simultaneously evaluated by measuring the luminescence spectrum in the culture medium without quantifying by a method such as electrophoresis.

## Claims

1. A monitor protein capable of measuring processing of a protein, comprising
- one or more processing cleavage regions containing an amino acid residue or an amino acid sequence to be processed; and
- one or more property variable regions, each comprising a luminescent protein and a fluorescent protein which exhibit changes in their luminescent and fluorescent energy properties upon said processing, **characterised in that**:
the luminescent protein is the *Cypridina noctiluca* secretory luciferase; and
the fluorescent protein is the luminescent *Aequorea victoria* yellow fluorescent protein.

2. A monitor protein according to claim 1 wherein a processing enzyme which cleaves the processing cleavage region is any one selected from the group consisting of PC1, PC2, furin, proteasome, cathepsin and thrombin.

3. A monitor protein according to claim 2 wherein the processing enzyme is PC1.

4. A monitor protein according to claim 1 wherein the processing cleavage region is located between the luminescent protein and the fluorescent protein which constitute the property variable region.

5. A monitor protein according to claim 1 wherein the processing cleavage region is composed of a sequence of 6 to 100 amino acids including a cleavage point cleaved by the processing enzyme.

6. A monitor protein according to claim 1 having the amino acid sequence represented in SEQ. ID NO. 2 or a mutant thereof encoded by a DNA sequence having at least 90% sequence homology to SEQ. ID NO. 1, wherein the mutant protein comprises:
one or more processing cleavage regions containing an amino acid residue or an amino acid sequence to be processed; and
one or more property variable regions, each comprising a luminescent protein and a fluorescent protein which exhibit changes in their luminescent and fluorescent energy properties upon said processing.

7. A monitor protein according to claim 1 wherein the processing cleavage region is SEQKQLQKRFGGFTGG.

8. DNA encoding the monitor protein according to any of claims 1 to 7.

9. DNA according to claim 8, having the sequence according to SEQ. ID NO. 1 or DNA which:
hybridizes to said sequence under stringency conditions of 1x SSC and 0.1 % SDS at 37 degrees C; or
has at least 90% sequence homology with SEQ ID NO. 1, wherein the DNA encodes a protein comprising:
one or more processing cleavage regions containing an amino acid residue or an amino acid sequence to be processed; and
one or more property variable regions, each comprising a luminescent protein and a fluorescent protein which exhibit changes in their luminescent and fluorescent energy properties upon said processing.

10. An expression vector comprising the DNA according to claim 8.

11. A method for measuring a processing ability of a certain cell **characterized by** introducing any of the monitor protein according to any of claims 1 to 7, the DNA according to claim 8 to 9 and the expression vector according to claim 10 into the cell, and quantitatively evaluating a change in energy transfer property of the monitor protein.

12. A method according to claim 16 wherein said cell is a cell derived from human.

13. A method for measuring a processing ability of a subject protein comprising a step of reacting the subject protein with the monitor protein according to any of claims 1 to 7 and a step of measuring a change in energy transfer property of the monitor protein.

14. A method for screening a processing enzyme, comprising a step of reacting a subject protein with the monitor protein according to any of claims 1 to 7 and a step of selecting the subject protein which changes a property of the monitor protein by measuring a change in energy transfer property of the monitor protein before and after the reaction with the subject protein.

15. A method for screening a compound which facilitates or inhibits processing, comprising a step of contacting a subject sample and a processing enzyme and the monitor protein according to any of claims 1 to 7 and a step of selecting a sample which facilitates or inhibits the property by measuring a change in energy transfer property of the monitor protein before and after the contact with the subject sample.

16. A method for screening a compound which facilitates or inhibits processing (enzyme activity), comprising a step of preparing a cell into which any of the monitor protein according to any of claims 1 to 7, the DNA according to claims 8 to 9 and the expression vector according to claim 10 is introduced, and a step of selecting a sample which facilitates or inhibits a property change by measuring the property change in energy transfer of the monitor protein expressed in the cell in the presence or absence of the subject sample.

## Patentansprüche

1. Überwachungsprotein, fähig zum Messen der Verarbeitung eines Proteins, umfassend:
- eine oder mehrere Verarbeitungsspaltungsregionen, enthaltend einen Aminosäurerest oder eine Aminosäuresequenz, die verarbeitet werden sollen; und
- eine oder mehrere eigenschaftsvariable Regionen, jede umfassend ein lumineszierendes Protein und ein fluoreszierendes Protein, welche bei der Verarbeitung Veränderungen in ihren Lumineszenz- und Fluoreszenzenergie-Eigenschaften zeigen, **dadurch gekennzeichnet, daß**:
das lumineszierende Protein die sekretorische Luciferase von *Cypridina noctiluca* ist; und
das fluoreszierende Protein das gelb fluoreszierende Protein von lumineszierender *Aequorea victoria* ist.

2. Überwachungsprotein gemäß Anspruch 1, wobei ein Verarbeitungsenzym, welches die Verarbeitungsspaltungsregion spaltet, eines, ausgewählt aus der Gruppe, bestehend aus PC1, PC2, Furin, Proteasom, Cathepsin und Thrombin, ist.

3. Überwachungsprotein gemäß Anspruch 2, wobei das Verarbeitungsenzym PC1 ist.

4. Überwachungsprotein gemäß Anspruch 1, wobei die Verarbeitungsspaltungsregion sich zwischen dem lumineszierenden Protein und dem fluoreszierenden Protein befindet, welche die eigenschaftsvariable Region ausmachen.

5. Überwachungsprotein gemäß Anspruch 1, wobei die Verarbeitungsspaltungsregion aus einer Sequenz von 6 bis 100 Aminosäuren einschließend einen Spaltungspunkt, gespalten durch das Verarbeitungsenzym, besteht.

6. Überwachungsprotein gemäß Anspruch 1 mit der Aminosäuresequenz, dargestellt in SEQ. ID NO. 2, oder eine Mutante davon, codiert durch eine DNA-Sequenz mit mindestens 90% Sequenzhomologie zu SEQ. ID NO. 1, wobei das mutante Protein umfaßt:
eine oder mehrere Verarbeitungsspaltungsregionen, enthaltend einen Aminosäurerest oder eine Aminosäuresequenz, die verarbeitet werden sollen, und
eine oder mehrere eigenschaftsvariable Regionen, jede umfassend ein lumineszierendes Protein und ein fluoreszierendes Protein, welche bei der Verarbeitung Veränderungen in ihren Lumineszenz- und Fluoreszenzenergie-Eigenschaften zeigen.

7. Überwachungsprotein gemäß Anspruch 1, wobei die Verarbeitungsspaltungsregion SEQKQLQKRFGGFTGG ist.

8. DNA, codierend das Überwachungsprotein gemäß einem der Ansprüche 1 bis 7.

9. DNA gemäß Anspruch 8 mit der Sequenz gemäß SEQ. ID NO. 1 oder DNA, welche:
mit der Sequenz unter Stringenzbedingungen von 1 x SSC und 0,1% SDS bei 37 Grad C hybridisiert; oder
mindestens 90% Sequenzhomologie mit SEQ. ID NO. 1 hat, wobei die DNA ein Protein codiert, umfassend:
eine oder mehrere Verarbeitungsspaltungsregionen, enthaltend einen Aminosäurerest oder eine Aminosäuresequenz, die verarbeitet werden sollen, und
eine oder mehrere eigenschaftsvariable Regionen, jede umfassend ein lumineszierendes Protein und ein fluoreszierendes Protein, welche bei der Verarbeitung Veränderungen in ihren Lumineszenz- und Fluoreszenzenergie-Eigenschaften zeigen.

10. Expressionsvektor, umfassend die DNA gemäß Anspruch 8.

11. Verfahren zum Messen einer Verarbeitungsfähigkeit einer bestimmten Zelle, **gekennzeichnet durch** Einführen eines Überwachungsproteins gemäß einem der Ansprüche 1 bis 7, der DNA gemäß Anspruch 8 oder 9 und des Expressionsvektors gemäß Anspruch 10 in die Zelle und quantitatives Bewerten einer Veränderung in der Energieübertragungseigenschaft des Überwachungsproteins.

12. Verfahren gemäß Anspruch 11, wobei die Zelle eine Zelle, abgeleitet von einem Menschen, ist.

13. Verfahren zum Messen einer Verarbeitungsfähigkeit eines betreffenden Proteins, umfassend einen Schritt, das betreffende Protein mit dem Überwachungsprotein gemäß einem der Ansprüche 1 bis 7 umzusetzen, und einen Schritt, eine Veränderung in der Energieübertragungseigenschaft des Überwachungsproteins zu messen.

14. Verfahren zum Screening eines Verarbeitungsenzyms, umfassend einen Schritt, ein betreffendes Protein mit dem Überwachungsprotein gemäß einem der Ansprüche 1 bis 7 umzusetzen, und einen Schritt, das betreffende Protein, welches eine Eigenschaft des Überwachungsproteins verändert, durch Messen einer Veränderung in der Energieübertragungseigenschaft des Überwachungsproteins vor und nach der Reaktion mit dem betreffenden Protein auszuwählen.

15. Verfahren zum Screening einer Verbindung, welche die Verarbeitung erleichtert oder hemmt, umfassend einen Schritt, eine betreffende Probe und ein Verarbeitungsenzym und das Überwachungsprotein gemäß einem der Ansprüche 1 bis 7 in Kontakt zu bringen, und einen Schritt, eine Probe, welche die Eigenschaft erleichtert oder hemmt, durch Messen einer Veränderung in der Energieübertragungseigenschaft des Überwachungsproteins vor und nach dem Kontakt mit der betreffenden Probe auszuwählen.

16. Verfahren zum Screening einer Verbindung, welche die Verarbeitung (Enzymaktivität) erleichtert oder hemmt, umfassend einen Schritt, eine Zelle herzustellen, in welche ein Überwachungsprotein gemäß einem der Ansprüche 1 bis 7, die DNA gemäß den Ansprüchen 8 und 9 und der Expressionsvektor gemäß Anspruch 10 eingeführt wird, und einen Schritt, eine Probe, welche eine Eigenschaftsänderung erleichtert oder hemmt, durch Messen der Eigenschaftsänderung in der Energieübertragung des Überwachungsproteins, exprimiert in der Zelle in Gegenwart oder Abwesenheit der betreffenden Probe, auszuwählen.

## Revendications

1. Protéine de surveillance, capable de mesurer le traitement d'une protéine, comprenant:
- une ou plusieurs régions de traitement par clivage contenant un résidu d'acide aminé ou une séquence d'acides aminés à traiter; et
- une ou plusieurs régions à propriété variable, chacune comprenant une protéine luminescente et une protéine fluorescente qui affichent des changements dans leurs propriétés d'énergies luminescente et fluorescente lors dudit traitement, **caractérisées en ce que**:
la protéine luminescente est la luciférase sécrétoire de *Cypridina noctiluca*; et
la protéine fluorescente est la protéine fluorescente jaune de *Aequorea victoria* luminescente.

2. Protéine de surveillance selon la revendication 1, dans laquelle une enzyme de traitement qui clive la région de traitement par clivage est n'importe laquelle choisie dans le groupe constitué de PC1, de PC2, de furine, de protéasome, de cathépsine et de thrombine.

3. Protéine de surveillance selon la revendication 2, dans laquelle l'enzyme de traitement est PC 1.

4. Protéine de surveillance selon la revendication 1, dans laquelle la région de traitement par clivage est localisée entre la protéine luminescente et la protéine fluorescente qui constituent la région à propriété variable.

5. Protéine de surveillance selon la revendication 1, dans laquelle la région de traitement par clivage est composée d'une séquence de 6 à 100 acides aminés incluant un point de clivage clivé par l'enzyme de traitement.

6. Protéine de surveillance selon la revendication 1, possédant la séquence d'acides aminés représentée dans la SEQ. ID NO. 2 ou un mutant de celle-ci codée par une séquence d'ADN possédant au moins 90% d'homologie de séquence avec la SEQ. ID NO. 1, dans laquelle la protéine mutante comprend:
une ou plusieurs régions de traitement par clivage contenant un résidu d'acide aminé ou une séquence d'acides aminés à traiter; et
une ou plusieurs régions à propriété variable, chacune comprenant une protéine luminescente et une protéine fluorescente qui affichent des changements dans leurs propriétés d'énergies luminescente et fluorescente lors dudit traitement.

7. Protéine de surveillance selon la revendication 1, dans laquelle la région de traitement par clivage est SEQKQLQKRFGGFTGG.

8. ADN codant pour la protéine de surveillance selon l'une quelconque des revendications 1 à 7.

9. ADN selon la revendication 8, possédant la séquence selon la SEQ. ID NO. 1 ou ADN qui:
s'hybride avec ladite séquence sous des conditions strictes de 1 x SSC et de 0,1% de SDS à 37 degrés C; ou
possède au moins 90% d'homologie de séquence avec la SEQ. ID NO. 1, où l'ADN code pour une protéine comprenant:
une ou plusieurs régions de traitement par clivage contenant un résidu d'acide aminé ou une séquence d'acides aminés à traiter; et
une ou plusieurs régions à propriété variable, chacune comprenant une protéine luminescente et une protéine fluorescente qui affichent des changements dans leurs propriétés d'énergies luminescente et fluorescente lors dudit traitement.

10. Vecteur d'expression comprenant l'ADN selon la revendication 8.

11. Procédé pour la mesure de la capacité de traitement d'une certaine cellule **caractérisé par** l'introduction de n'importe laquelle de la protéine de surveillance selon l'une quelconque des revendications 1 à 7, de l'ADN selon les revendications 8 à 9 et du vecteur d'expression selon la revendication 10 dans la cellule, et l'évaluation quantitative d'un changement dans la propriété de transfert d'énergie de la protéine de surveillance.

12. Protéine selon la revendication 11, dans lequel ladite cellule est une cellule dérivée d'un humain.

13. Procédé pour la mesure de la capacité de traitement d'une cellule sujet comprenant une étape de réaction de la protéine sujet avec la protéine de surveillance selon l'une quelconque des revendications 1 à 7 et une étape de mesure d'un changement dans la propriété de transfert d'énergie de la protéine de surveillance.

14. Procédé pour le criblage d'une enzyme de traitement, comprenant une étape de réaction d'une protéine sujet avec la protéine de surveillance selon l'une quelconque des revendications 1 à 7 et une étape de sélection de la protéine sujet qui change une propriété de la protéine de surveillance en mesurant un changement dans la propriété de transfert d'énergie de la protéine de surveillance avant et après la réaction avec la protéine sujet.

15. Procédé pour le criblage d'un composé qui facilite ou inhibe un traitement, comprenant une étape de mise en contact d'un échantillon sujet et d'une enzyme de traitement et de la protéine de surveillance selon l'une quelconque des revendications 1 à 7 et une étape de sélection d'un échantillon qui facilite ou inhibe la propriété en mesurant un changement dans la propriété de transfert d'énergie de la protéine de surveillance avant et après le contact avec l'échantillon sujet.

16. Procédé pour le criblage d'un composé qui facilite ou inhibe un traitement (activité d'enzyme) comprenant une étape de préparation d'une cellule dans laquelle n'importe laquelle de la protéine de surveillance selon l'une quelconque des revendications 1 à 7, l'ADN selon les revendications 8 à 9 et le vecteur d'expression selon la revendication 10 sont introduits, et une étape de sélection d'un échantillon qui facilite ou inhibe un changement de propriété en mesurant le changement de propriété dans le transfert d'énergie de la protéine de surveillance exprimée dans la cellule en présence ou en l'absence de l'échantillon sujet.


### SEQUENCE LISTING

<110> JAPAN SCIENCE AND TECHNOLOGY AGENCY
<120> Monitor protein for measuring protein processing
<130> EPP92674
<150> JP2002-360744
<151> 2002-12-12
<160> 5
<170> PatentIn version 3.1
<210> 1
   <211> 2052
   <212> DNA
   <213> mammalian
   <400> 1
<210> 2
   <211> 833
   <212> PRT
   <213> mammalian
<400> 2
   Met Lys Ile Ile Ile Leu Ser Val Ile Leu Ala Tyr Cys Val Thr Asp
   1               5                   10                  15
   Asn Cys Gln Asp Ala Cys Pro Val Glu Ala Glu Pro Pro Ser Ser Thr
               20                  25                  30
   Pro Thr Val Pro Thr Ser Cys Glu Ala Lys Glu Gly Glu Cys Ile Asp
           35                  40                  45
   Thr Arg Cys Ala Thr Cys Lys Arg Asp Ile Leu Ser Asp Gly Leu Cys
       50                  55                  60
   Glu Asn Lys Pro Gly Lys Thr Cys Cys Arg Met Cys Gln Tyr Val Ile
   65                  70                  75                  80
   Glu Cys Arg Val Glu Ala Ala Gly Tyr Phe Arg Thr Phe Tyr Gly Lys
                   85                  90                  95
   Arg Phe Asn Phe Gln Glu Pro Gly Lys Tyr Val Leu Ala Arg Gly Thr
   100 105 110
   Lys Gly Gly Asp Trp Ser Val Thr Leu Thr Met Glu Asn Leu Asp Gly
   115 120 125
   Gln Lys Gly Ala Val Leu Thr Lys Thr Thr Leu Glu Val Ala Gly Asp
   130 135 140
   Val Ile Asp Ile Thr Gln Ala Thr Ala Asp Pro Ile Thr Val Asn Gly
   145 150 155 160
   Gly Ala Asp Pro Val Ile Ala Asn Pro Phe Thr Ile Gly Glu Val Thr
   165 170 175
   Ile Ala Val Val Glu Ile Pro Gly Phe Asn Ile Thr Val Ile Glu Phe
   180 185 190
   Phe Lys Leu Ile Val Ile Asp Ile Leu Gly Gly Arg Ser Val Arg Ile
   195 200 205
   Ala Pro Asp Thr Ala Asn Lys Gly Leu Ile Ser Gly Ile Cys Gly Asn
   210 215 220
   Leu Glu Met Asn Asp Ala Asp Asp Phe Thr Thr Asp Ala Asp Gln Leu
   225 230 235 240
   Ala Ile Gln Pro Asn Ile Asn Lys Glu Phe Asp Gly Cys Pro Phe Tyr
   245 250 255
   Gly Asn Pro Ser Asp Ile Glu Tyr Cys Lys Gly Leu Met Glu Pro Tyr
   260 265 270
   Arg Ala Val Cys Arg Asn Asn Ile Asn Phe Tyr Tyr Tyr Thr Leu Ser
   275 280 285
   Cys Ala Phe Ala Tyr Cys Met Gly Gly Glu Glu Arg Ala Lys His Val
   290 295 300
   Leu Phe Asp Tyr Val Glu Thr Cys Ala Ala Pro Glu Thr Arg Gly Thr
   305 310 315 320
   Cys Val Leu Ser Gly His Thr Phe Tyr Asp Thr Phe Asp Lys Ala Arg
   325 330 335
   Tyr Gln Phe Gln Gly Pro Cys Lys Glu Ile Leu Met Ala Ala Asp Cys
   340 345 350
   Tyr Trp Asn Thr Trp Asp Val Lys Val Ser His Arg Asp Val Glu Ser
   355 360 365
   Tyr Thr Glu Val Glu Lys Val Thr Ile Arg Lys Gln Ser Thr Val Val
       370                 375                 380
   Asp Leu Ile Val Asp Gly Lys Gln Val Lys Val Gly Gly Val Asp Val
   385                 390                 395                 400
   Ser Ile Pro Tyr Ser Ser Glu Asn Thr Ser Ile Tyr Trp Gln Asp Gly
                   405                 410                 415
   Asp Ile Leu Thr Thr Ala Ile Leu Pro Glu Ala Leu Val Val Lys Phe
               420                 425                 430
   Asn Phe Lys Gln Leu Leu Val Val His Ile Arg Asp Pro Phe Asp Gly
           435                 440                 445
   Lys Thr Cys Gly Ile Cys Gly Asn Tyr Asn Gln Asp Ser Thr Asp Asp
       450                 455                 460
   Phe Phe Asp Ala Glu Gly Ala Cys Ala Leu Thr Pro Asn Pro Pro Gly
   465                 470                 475                 480
   Cys Thr Glu Glu Gln Lys Pro Glu Ala Glu Arg Leu Cys Asn Asn Leu
                   485                 490                 495
   Phe Asp Ser Ser Ile Asp Glu Lys Cys Asn Val Cys Tyr Lys Pro Asp
               500                 505                 510
   Arg Ile Ala Arg Cys Met Tyr Glu Tyr Cys Leu Arg Gly Gln Gln Gly
           515                 520                 525
   Phe Cys Asp His Ala Trp Glu Phe Lys Lys Glu Cys Tyr Ile Lys His
       530                 535                 540
   Gly Asp Thr Leu Glu Val Pro Pro Glu Cys Gln Gly Ser Thr Glu Pro
   545                 550                 555                 560
   Gly Leu Glu Glu Val Gly Glu Ile Glu Gln Lys Gln Leu Gln Lys Arg
                   565                 570                 575
   Phe Gly Gly Phe Thr Gly Ala Arg Lys Ser Ala Arg Lys Leu Ala Asn
               580                 585                 590
   Gln Gly Ser Val Ser Lys Gly Glu Glu Leu Phe Thr Gly Val Val Pro
           595                 600                 605
   Ile Leu Val Glu Leu Asp Gly Asp Val Asn Gly His Lys Phe Ser Val
       610                 615                 620
   Ser Gly Glu Gly Glu Gly Asp Ala Thr Tyr Gly Lys Leu Thr Leu Lys
   625                 630                 635                 640
   Phe Ile Cys Thr Thr Gly Lys Leu Pro Val Pro Trp Pro Thr Leu Val
   645 650 655
   Thr Thr Phe Gly Tyr Gly Leu Gln Cys Phe Ala Arg Tyr Pro Asp His
   660 665 670
   Met Lys Gln His Asp Phe Phe Lys Ser Ala Met Pro Glu Gly Tyr Val
   675 680 685
   Gln Glu Arg Thr Ile Phe Phe Lys Asp Asp Gly Asn Tyr Lys Thr Arg
   690 695 700
   Ala Glu Val Lys Phe Glu Gly Asp Thr Leu Val Asn Arg Ile Glu Leu
   705 710 715 720
   Lys Gly Ile Asp Phe Lys Glu Asp Gly Asn Ile Leu Gly His Lys Leu
   725 730 735
   Glu Tyr Asn Tyr Asn Ser His Asn Val Tyr Ile Met Ala Asp Lys Gln
   740 745 750
   Lys Asn Gly Ile Lys Val Asn Phe Lys Ile Arg His Asn Ile Glu Asp
   755 760 765
   Gly Ser Val Gln Leu Ala Asp His Tyr Gln Gln Asn Thr Pro Ile Gly
   770 775 780
   Asp Gly Pro Val Leu Leu Pro Asp Asn His Tyr Leu Ser Tyr Gln Ser
   785 790 795 800
   Ala Leu Ser Lys Asp Pro Asn Glu Lys Arg Asp His Met Val Leu Leu
   805 810 815
   Glu Phe Val Thr Ala Ala Gly Ile Thr Leu Gly Met Asp Glu Leu Tyr
   820 825 830
   Lys
<210> 3
   <211> 16
   <212> PRT
   <213> mammalian
<400> 3
   Ser Glu Gln Lys Gln Leu Gln Lys Arg Phe Gly Gly Phe Thr Gly Gly
   1 5 10 15
<210> 4
   <211> 16
   <212> PRT
   <213> mammalian
<400> 4
   Ser Glu Gln Lys Gln Leu Gln Gly Gly Phe Gly Gly Phe Thr Gly Gly
   1               5                   10                  15
<210> 5
   <211> 14
   <212> PRT
   <213> mammalian
<400> 5
   Ser Glu Gln Lys Gln Leu Gln Phe Gly Gly Phe Thr Gly Gly
   1               5                   10